# EUROPEAN PATENT APPLICATION

(11) **EP 2 182 340 A1**
(43) Date of publication of application: **05.05.2010**
(21) Application number: 08167971.4
(22) Date of filing: 30.10.2008
(51) Int. Cl.: G01L 9/00, G01L 19/00

(54) **Pressure Sensor and Guide Wire Assembly**

(71) Applicant: Radi Medical Systems AB, 75135 Uppsala (SE)
(72) Inventor: Tiensuu, Stefan, SE-75652, UPPSALA (SE); Rydberg, Matts, SE-75756, UPPSALA (SE); Jönsson, Mats, SE-75318, UPPSALA (SE)

(57) **Abstract**

A pressure sensor chip is described. The pressure sensor chip include a substrate, a polycrystalline silicon layer, at least one silicon layer, and a diaphragm movement element. The polycrystalline silicon layer is formed on the substrate and has a cavity recess formed therein. The at least one silicon layer is formed on the polycrystalline silicon layer and covers the cavity recess thereby forming a reference chamber with a diaphragm. The diaphragm movement element is configured to sense movement of the diaphragm. An assembly incorporating the pressure sensor chip and a method of forming the pressure sensor chip are also described.

## Description

### Field of the invention

The present invention relates generally to pressure sensors used in the medical field and in particular to such sensors used in situ to measure intracoronary pressure and mounted at the distal end of a guide wire, and to methods of manufacture of such sensors.

### Background of the Invention

In order to determine or assess the ability of a specific coronary vessel to supply blood to the heart muscle, i.e. the myocardium, there is known a method by which the intracoronary pressure distally of a stenosis in combination with the proximal pressure is measured. The method is a determination of the so-called Fractional Flow Reserve (See "Fractional Flow Reserve", Circulation, Vol. 92, No. 11, Dec. 1, 1995, by Nico H. j. Pijls et al.). Briefly, FFR_{myo} is flow defined as the ratio between the pressure distally of a stenosis and the pressure proximally of a stenosis, i.e. FFR_{myo}=P_{dist}/Pₚᵣₒₓ. The distal pressure is measured in the vessel using a micro-pressure transducer, and the proximal pressure is either the arterial pressure or measured with the same transducer after pulling it back to a position proximal of the stenosis.

One arrangement that could be used in measuring FFR is a sensor guide having a sensor element, an electronic unit, a signal transmitting cable connecting the sensor element to the electronic unit, a flexible tube having the sensor element and cable disposed therein, a solid metal wire having a plurality of sections such that each of the sections has a different flexibility, and a coil which is attached to the distal end of the wire. Examples of such sensor guide wire assemblies are described in U.S. Patent Nos. 6,112,598, RE35,648 and 6,167,763, where the contents of these patents are hereby incorporated for the assemblies and methods described therein.

Pressure sensors used in the context of measuring intracoronary pressure often contain a deflectable diaphragm. The two main types of such pressure sensors are absolute pressure sensors and differential or relative pressure sensors. In an absolute pressure sensor the diaphragm is usually mounted across a small cavity wherein a reference pressure, usually vacuum pressure, exists, and the pressure to be measured acts on the opposing surface of the diaphragm. A differential pressure sensor measures the difference of two pressures acting on opposing sides of the diaphragm.

The movement or deformation of the diaphragm can be sensed in different ways, such as by measuring the changes of electric characteristics of a piezoresistive body, the changes of resistance of an electrical conductor or the change of capacitance of a suitable adapted capacitor coupled to the movement of the diaphragm and thereby being in varied forced or strained states.

Absolute pressure sensors need a hermetic sealing of a relatively small cavity at the active diaphragm to get a reference pressure, preferably a vacuum enclosure. This can be accomplished on a wafer using e.g. silicon wafer bonding under vacuum conditions.

Generally, for example for use in a sensor guide wire assembly as described above, a small piezoresistive absolute pressure sensor is desired, having a high pressure sensitivity, a controlled temperature behavior and a high long term stability. It should not be affected by environmental changes, such as humidity or possible temperature fluctuations. Also, a manufacturing process suitable for high volume production and with a high yield is preferred.

Recently, micromachining techniques have been developed and refined for producing integrated miniaturized pressure sensors of semiconductor material, providing several advantages over traditional pressure sensors: low cost, high degree of performance and reliability, better signal/noise ratio, and greater reproducibility.

Several pressure sensors based on silicon-on-insulator (SOI) substrates have been proposed. For example, U.S. Patent Nos. 6,131,466, 5,510,276, 5,095,401, and 7,207,227, disclose such sensors. In U.S. Patent No. 7,207,227, a method of manufacturing a pressure sensor is described, wherein a cavity is formed in an SOI substrate, and thereafter a second silicon wafer is bonded to the first to seal the cavity. After several etching and deposition steps, a sensor complete with electrical strain gauge is produced.

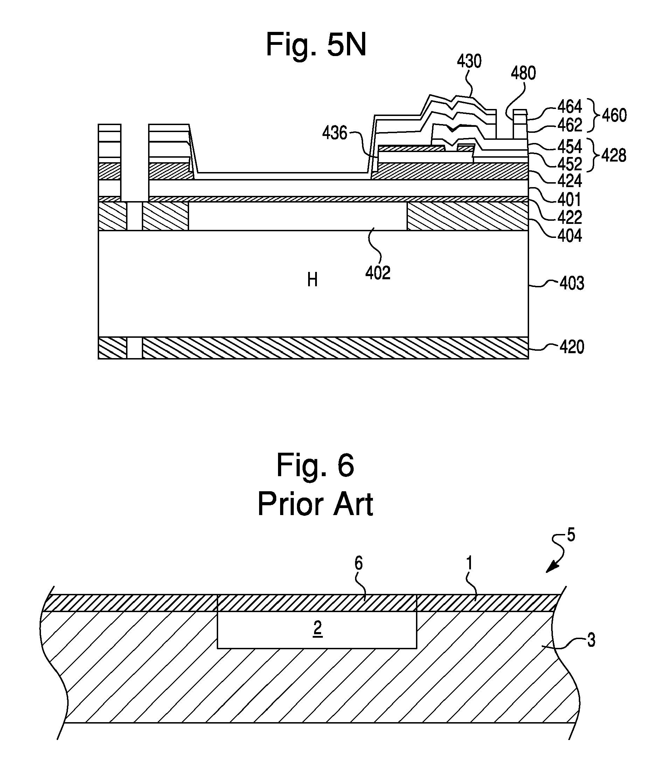

FIG. 6 is a schematic illustrating a conventional pressure sensor chip 5 based on an SOI substrate. The pressure sensor chip 5 includes a crystalline silicon substrate 3, a cavity recess 2 formed in the crystalline silicon substrate 3, and a crystalline silicon layer 1 bonded to the crystalline silicon substrate 3 and covering the cavity recess 2. The crystalline silicon layer 1 has a diaphragm 6 formed over the cavity recess 2. A certain pressure exerted on the diaphragm 6 from the surrounding medium will thereby correspond to a certain stretching of the diaphragm 6 and thereby to a certain resistance of piezoresistive elements (not shown in FIG. 6), disposed on the membrane.

### Summary of the Invention

According to one embodiment of the invention there is provided a pressure sensor chip.
The pressure sensor chip comprises: a substrate; a polycrystalline silicon layer formed on the substrate and having a cavity recess formed therein; at least one silicon layer formed on the polycrystalline silicon layer and covering the cavity recess thereby forming a reference chamber with a diaphragm; and a diaphragm movement element configured to sense movement of the diaphragm.

According to another embodiment of the invention there is provided a pressure sensor and guide wire assembly. The pressure sensor and guide wire assembly comprises: a sensor chip; a wire; and a mount, wherein the sensor chip is mounted to the wire via the mount. The sensor chip comprises: a substrate; a polycrystalline silicon layer formed on the substrate and having a cavity recess formed therein; at least one silicon layer formed on the polycrystalline silicon layer and covering the cavity recess thereby forming a reference chamber with a diaphragm; and a diaphragm movement element configured to sense movement of the diaphragm.

According to another embodiment of the invention there is provided a method of forming a pressure sensor chip. The method comprises: providing a substrate; forming a polycrystalline silicon layer on the substrate; forming a cavity recess in the polycrystalline silicon layer; bonding at least one silicon layer to the polycrystalline silicon layer to cover the cavity recess thereby forming a reference chamber with a diaphragm; and forming a diaphragm movement element configured to sense movement of the diaphragm.

### Brief Description of the Drawings

FIG. 1 is a schematic illustrating a pressure sensor chip according to an embodiment of the invention.
FIG. 2 is a schematic illustrating a pressure sensor including the pressure sensor chip of FIG. 1, according to an embodiment of the invention.
FIG. 3 illustrates, in longitudinal cross-section, a pressure sensor and guide wire assembly including the pressure sensor chip of FIG. 1, according to an embodiment of the invention.
FIG. 4 illustrates, in cross-section, a pressure sensor chip according to an embodiment of the invention.
FIGs. 5A-5N illustrate steps in forming the pressure sensor chip of FIG. 4, according to an embodiment of the invention.
FIG. 6 is a schematic illustrating a conventional pressure sensor chip.

### Detailed Description of Preferred Embodiments

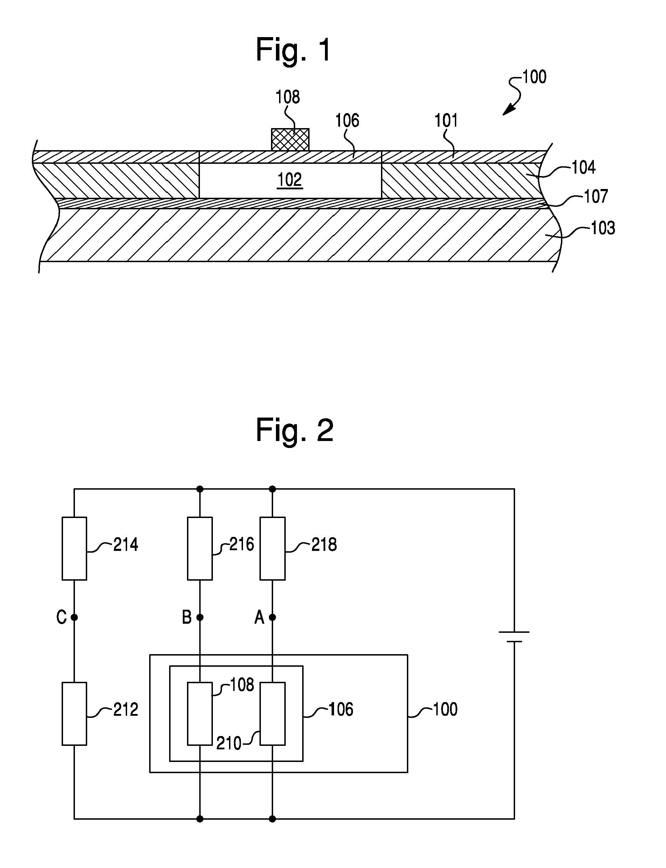

FIG. 1 illustrates a schematic of a pressure sensor chip 100 according to an embodiment of the invention. It should be noted that in producing the pressure sensor chip 100, a multitude of identical structures are generally produced, although only one structure is illustrated for ease in explanation. The pressure sensor chip 100 includes a substrate 103, such as semiconductor substrate, and in particular a crystalline silicon substrate, a polycrystalline silicon layer 104 formed on the substrate 103, a cavity recess 102 formed in the polycrystalline silicon layer 104, and a crystalline silicon layer 101 bonded to the polycrystalline silicon layer 104 and covering the cavity recess 102. Preferably, the substrate 103 is suitable for processing in silicon standard planar processing. The crystalline silicon layer 101 has a diaphragm 106 region formed over the cavity recess 102 thus forming a reference chamber. An optional etch stop layer 107 may be formed as a top surface of the substrate 103 under the polycrystalline silicon layer 104. In general, the sensor chip 100 may be configured to be an absolute pressure sensor chip or a differential pressure chip according to the medium provided in the reference chamber.

A certain pressure exerted on the diaphragm 106 from the surrounding medium will thereby correspond to a certain stretching of the diaphragm 106 and thereby to a certain electronic property response of a diaphragm movement element 108 formed on the diaphragm 106 due to the strain of the diaphragm movement element 108 with the stretching. The diaphragm movement element 108 is configured to sense movement of the diaphragm 106. The diaphragm movement element 108 may be, for example, one or more piezoresistive elements, capacitive elements, or a mechanically resonating element, for example. In the pressure sensor chip 100 of FIG. 1, unlike the conventional pressure sensor of FIG. 6, the cavity recess is formed in a polycrystalline silicon layer 104, which is formed on the substrate 103.

The disposition of the polycrystalline silicon layer 104 on the substrate 103, where the cavity recess 102 is formed in the polycrystalline silicon layer 104 instead of the substrate 103 provides advantages over the conventional structure shown in FIG. 6. First, the cavity recess 102 in the polycrystalline silicon layer 104 may be formed with more accurate and reproducible dimensions than is possible when there is no polycrystalline silicon layer 104 and the recess 102 is formed in the substrate. Second, bonding of the crystalline silicon layer 101 to the underlying polycrystalline silicon layer 104 is improved over bonding where merely a crystalline silicon substrate 103 is employed in the device.

The cavity recess 102 in the polycrystalline silicon layer 104 may be formed with more accurate and reproducible dimensions because the etching process can be tailored to be more accurate. In the case where the sensor chip 100 includes an etch stop layer 107 between the silicon substrate 103 and the polycrystalline silicon layer 104, an appropriate etchant is employed to provide that the polycrystalline silicon layer 104 is selectively etched relative to the etch stop 107, and the cavity recess 102 may be formed so as to expose the etch stop layer 107. Thus, the cavity recess 102 may be formed with an accurately controlled depth and volume. The particular etchant will depend on the material of the etch stop chosen. Alternatively, if no etch stop layer 107 is included, an etchant which selectively etches polycrystalline silicon relative to substrate 103 may be used, and the cavity recess 102 may be formed so as to expose the substrate 103. Suitable etching would include, for example, Deep Reactive Ion Etching (DRIE) using SF₆, or wet etching using KOH (Kalium hydroxide).

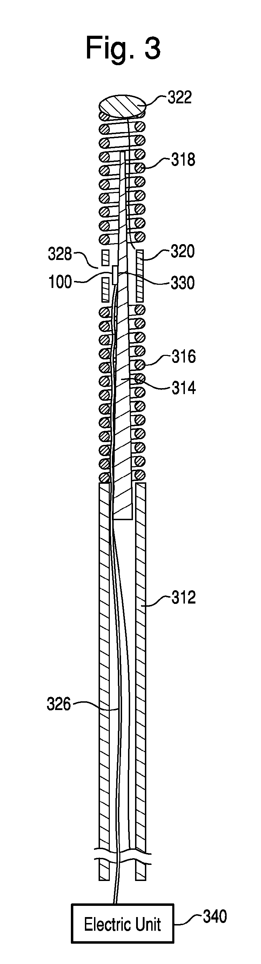

Forming the cavity recess 102 with accurate and reproducible dimensions is particularly important when the chip sensor 100 is employed as part of a pressure sensor and guide wire assembly (See assembly of FIG. 3, for example), where the chip sensor 100 may not be adequately tested and the assembly calibrated until the chip sensor is integrated as part of the assembly. Failure of the chip sensor 100 after integration can result in failure of the entire assembly. Thus, it is important to have a chip sensor where the cavity dimensions are accurate and reproducible.

Bonding is also improved when a polycrystalline layer is employed in the context of an SOI device. When forming the sensor chip 100, the crystalline silicon layer 101 can be formed when a silicon substrate is bonded to the underlying substrate having the cavity recess. The bonding is improved when the underlying substrate has a polycrystalline layer formed thereon, as compared with bonding directly to a crystalline silicon substrate.

In the case that the sensor chip includes an etch stop 107, there are many appropriate materials for the etch stop 107. Some examples of etch stop materials include carbon based material, nitrides, and oxides. Doping the top surface of the substrate 103 could also provide an etch stop layer.

FIG. 2 illustrates in a schematic fashion, a pressure sensor 200 including the pressure sensor chip 100, according to one embodiment of the invention. The sensor chip 100 includes piezoresistive element 108 and preferably a reference resistor 210 as the diaphragm movement element. The reference resistor 210 is preferably temperature sensitive, but not piezoresistive. The pressure sensor 200 further includes one Wheatstone bridge including the piezoresistive element 108 and another Wheatstone bridge including the reference resistor 210. Such a two Wheatstone bridge configuration with a piezoresistive element and a temperature sensitive reference resistor is described for, for example, in U.S. Reissued Patent RE39,863, which is incorporated by reference for its description of the two Wheatstone bridge configuration. Alternatively, the pressure sensor 200 may include the piezoresistive element 108 and associated Wheatstone bridge without the reference resistor 210 and its Wheatstone bridge.
As shown in FIG. 2, the piezoresistive element 108 and the reference resistor 210 are disposed on the diaphragm 106 of the sensor chip 100. Alternatively, the reference resistor 210 may be disposed on a portion of the sensor chip 100 not on the diaphragm 106. The pressure sensor in FIG. 2 further contains resistors 212, 214, 216 and 218. The first Wheatstone bridge comprises resistance element 108 and resistors 212, 214 and 216, while the second Wheatstone bridge comprises temperature sensitive reference resistor 210 and resistors 212, 214 and 218. Thus, resistors 212 and 214 are shared by the bridges. With the configuration shown in FIG. 2, it is possible to measure the temperature by measuring the current across points B and C, while the pressure can be determined by measuring the current across points A and C. The resistors 212, 214, 216 and 218 may be arranged external to the chip sensor 100 as shown in FIG. 2, or alternatively may be arranged on the chip sensor.

FIG. 3 illustrates a pressure sensor and guide wire assembly 300 according to one embodiment of the invention. The assembly 300 includes hollow tube 312, core wire 314, first coil 316, second coil 318, sleeve 320, dome-shaped tip 322, pressure sensor chip 100, and one or several electrical leads 326. An example of such a guide wire assembly, other than the pressure sensor chip 100, is shown in U.S. Patent No. 6,167,763, which is incorporated herein by reference for its disclosure of an assembly.

The proximal end of the first coil 316 is attached to the distal end of the hollow tube 312, while the distal end of the first coil 316 is attached to the proximal end of the sleeve 320. The proximal end of the second coil 318 is connected to the distal end of the jacket 320. Both the first and second coils 316, 318 are flexible coils, allowing flex in the assembly. The dome-shaped tip 322 is attached to the distal end of the second coil 318. The core wire 314 is at least partly disposed inside the hollow tube 312 such that the distal portion of the core wire 314 extends out of the hollow tube 312 and into the second coil 318.

The pressure sensor chip 100 is mounted on the core wire 314 at the position of the sleeve 320 via a mount 330. The pressure sensor chip 100 may be connected to an electronic unit 340 through the electrical leads 326. In the case that the sensor chip is deployed with a Wheatstone bridge configuration, such as that shown in FIG. 2, resistors of the Wheatstone bridge that are external to the sensor chip 100 may be included in the electrical unit 340. The electronic unit 340 may be part of the assembly 330, or external thereto. The assembly 300 also includes an aperture 328 in the sleeve 320, which in use allows the pressure sensor chip 100 to be in contact with a medium, such as blood, so that the pressure sensor chip 100 may measure the pressure of the medium.

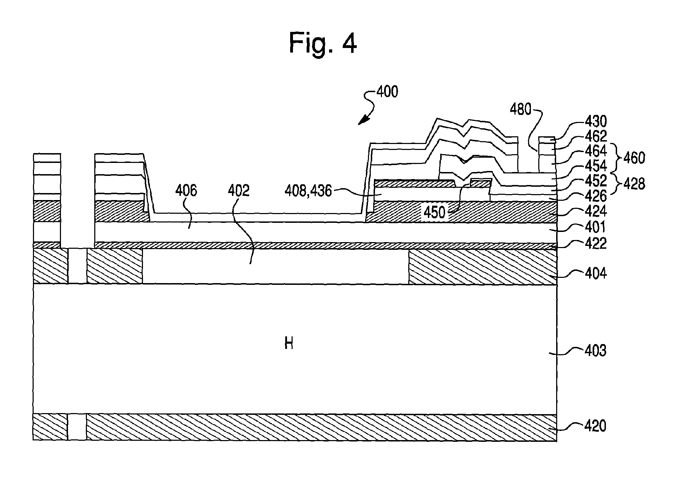

FIG. 4 illustrates a pressure sensor chip 400 according to an embodiment of the invention. The particular layer thicknesses for the chip 400 discussed below are exemplary only, and in general, ranges of thicknesses would be expected to be appropriate. The pressure sensor chip 400 includes a crystalline silicon wafer 403 with a polycrystalline layer formed on both sides thereof, namely a backside polycrystalline layer 420 and a polycrystalline layer 404. The backside polycrystalline layer 420 and the polycrystalline layer 404 may have thicknesses of about 1400 nm and 1300 nm, respectively, for example. The polycrystalline layer 404 has a cavity recess 402 formed therein.

The polycrystalline layer 404 is bonded to an overlying crystalline silicon layer 401 via bonding oxide layer 422. The crystalline silicon layer 401 and the bonding oxide layer 422 may have thicknesses of about 1500 nm and 20 nm, respectively, for example. The crystalline silicon layer 401 covers the cavity recess 402 thereby forming a reference chamber in the polycrystalline layer 404. The reference chamber may be filled with vacuum or a gas, as desired, and the sensor chip may be an absolute or differential pressure chip.
The region of the crystalline silicon layer 401 which is directly over the cavity recess 402 forms a diaphragm 406.

A diaphragm movement element 408 in the form of a piezoresistive layer 436 is formed on the crystalline silicon layer 401 at least party over the diaphragm 406. The piezoresistive layer 436 may have a thickness of about 400 nm, for example. The piezoresistive layer 436 may be of any appropriate piezoresistive material, such as doped silicon, for example. As the diaphragm is strained due to a difference in pressure within the reference chamber, and on the side of the diaphragm 406 away from the reference chamber, the resistive properties of the piezoresistive layer 436 are changed.

An insulator 424 layer, which may have a thickness of about 750 nm or 100 nm, for example, is formed between the piezoresistive layer 436 and the crystalline silicon layer 401 to form insulation therebetween. The insulator layer 424 may be any appropriate insulating material, such as nitrides, or oxides, for example, and may be a thermal oxide, for example. An insulator layer 426, which may have a thickness of about 200 nm, for example, is formed on the piezoresistive layer 436 between the piezoresistive layer 436 and overlying wiring layer 428, which contacts the piezoresistive layer 436 in a contact hole 450 in the insulator layer 426. The insulator layer 426 may be any appropriate insulating material, such as nitrides, or oxides, for example, and may be a TEOS oxide, for example.

The wiring layer 428 may comprise a conductor layer 454, or a conductor layer 454 and a barrier layer 452, where the barrier layer 452 is between the piezoresistive layer 436 and the conductor layer 454. The conductor layer 454 may be formed of an appropriate conducting material, such as aluminum or copper, for example, and may have a thickness of about 1100 nm, for example. The barrier layer may be any appropriate material which provides diffusion barrier properties between the piezoresistive layer 436 and the conductor layer 454, and may be a refractory metal or refractory metal compound, such as TiW or TiN, for example, and may have a thickness of about 50 nm, for example.

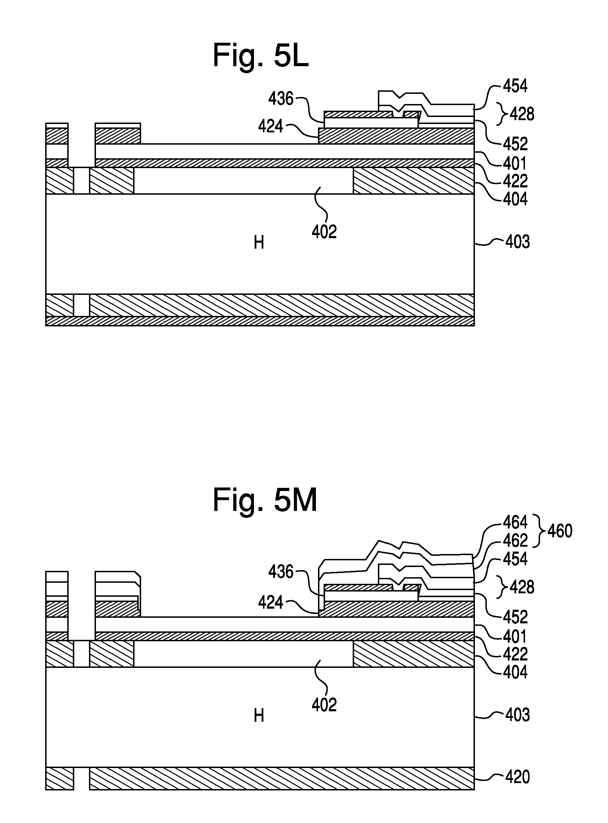

An overlying insulator layer 460 and passivating layer 470 may be formed over the wiring layer 428. The overlying insulator 460 may be of any appropriate insulating material, such as oxides or nitrides. For example, as shown in FIG. 5M, the overlying insulator 460 may be a bilayer of low temperature oxide (LTO) insulator 462 and silicon nitride insulator 464 with respective thickness of 700 nm and 650 nm, for example. The passivating layer 430 may be of any appropriate passivating material, such as oxides or nitrides, and may be silicon nitride for example, with an exemplary thickness of about 200 nm. A via hole 480 is provided in the passivating layer 430 and overlying insulator layer 460 down to the wiring layer 428 to provide access for an electrical contact to the wiring layer 428.

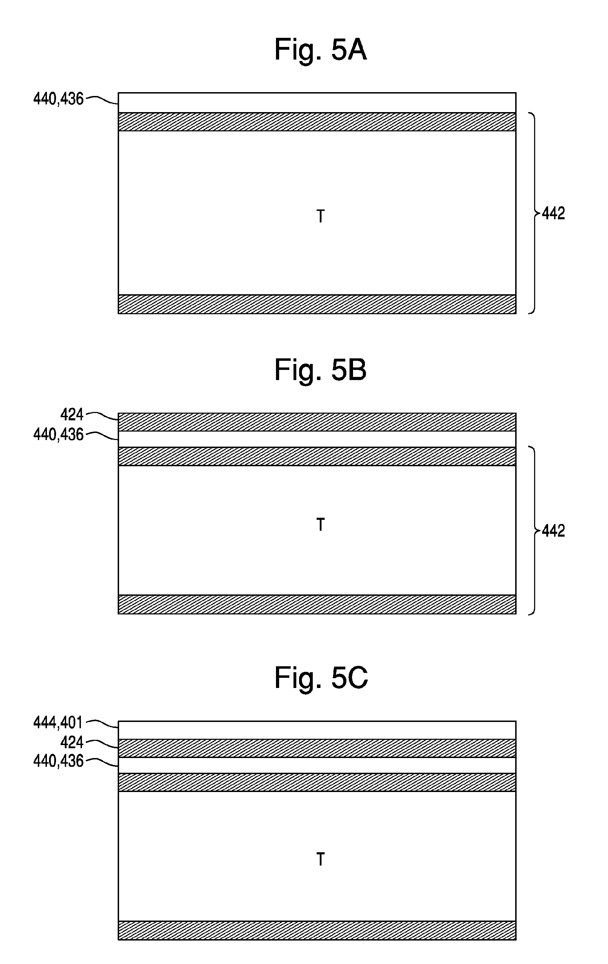

FIGs. 5A-5N illustrate a method for making a sensor chip, such as the chip illustrated in FIG. 4. A piezo wafer 440 of silicon is bonded to a temporary wafer substrate 442, and most of the piezo wafer 440 is then split off from the bonded structure to leave a piezoresistive layer 436 resulting in the bonded structure of FIG. 5A. The bonded structure of FIG. 5A is then polished to remove roughness of the piezoresistive layer 436, and the polished structure is thermally oxidized to produce insulator layer 424 of oxide as shown in FIG. 5B.

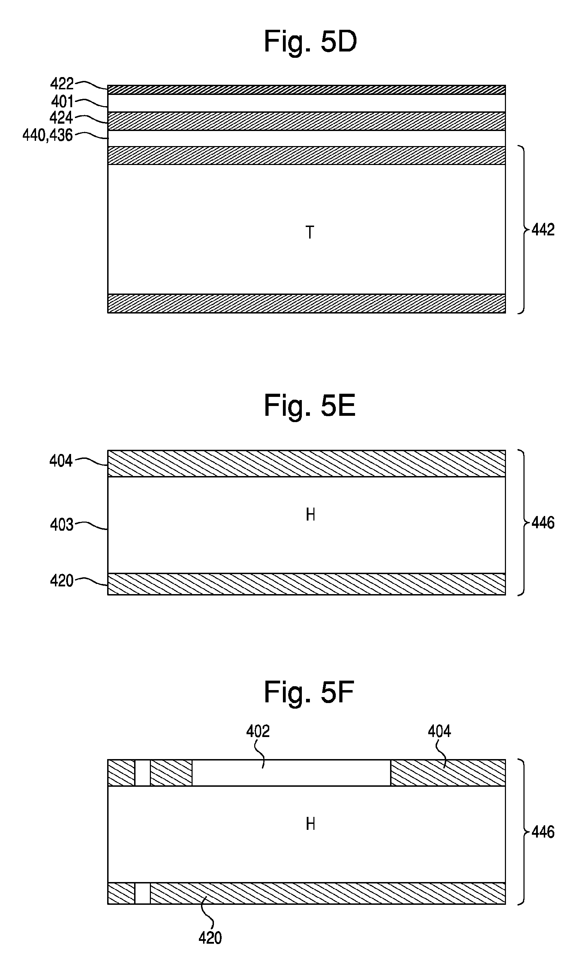

The bonded structure of FIG. 5B is then bonded to diaphragm wafer 444 and most of the diaphragm wafer material is then split off to leave crystalline silicon layer 401 resulting in the bonded structure shown in FIG. 5C. The bonded structure of FIG. 5C is then polished to remove roughness of the crystalline silicon layer 401, and the polished structure is thermally oxidized to produce bonding oxide 422 as shown in FIG. 5D.

A silicon substrate wafer 446 is processed by depositing polysilicon layers on both sides of the crystalline silicon substrate 403 resulting in the structure shown in FIG. 5E with polycrystalline layer 404 formed on one side of the crystalline silicon substrate 403, and backside polycrystalline layer 420 formed on the opposing side of the crystalline silicon substrate 403. Subsequently, as shown in FIG. 5F, a cavity recess 402 is patterned into the polycrystalline layer 404, such as by lithographic techniques including etching. Suitable etching would include, for example, Deep Reactive Ion Etching (DRIE) using SF₆, or wet etching using KOH (Kalium hydroxide).

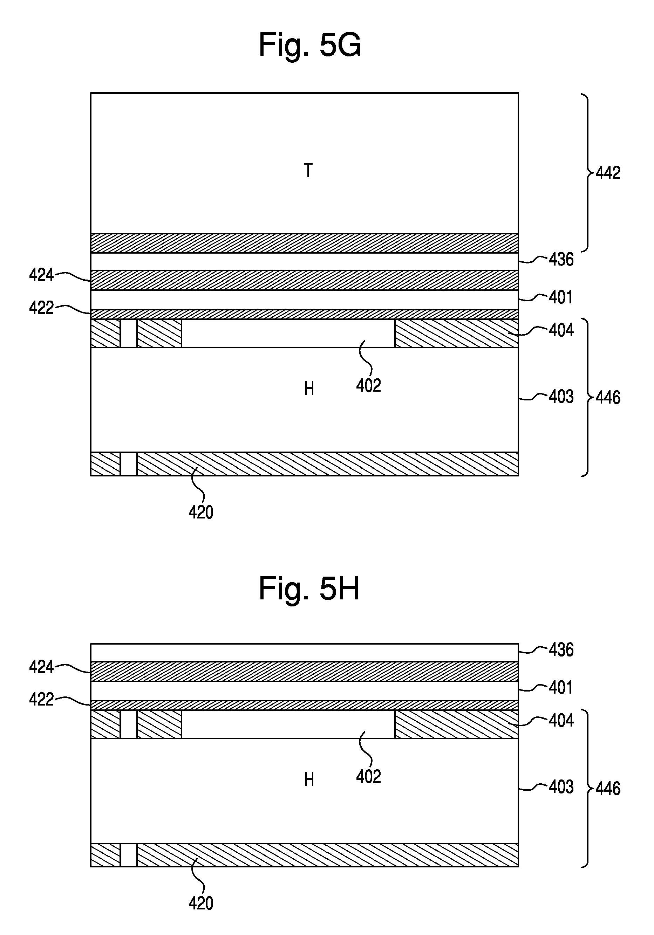

The silicon substrate wafer 446 with cavity recess 402 of FIG. 5F is then contacted with the wafer structure of 5D, where the polycrystalline layer 404 contacts the bonding oxide 422, and the structures are heated to be bonded to each other resulting in the bonded structure shown in FIG. 5G. The structure may be bonded, for example, using silicon fusing bonding. Beneficially, the polycrystalline layer 404 improves stress relief at the bonding interface. The temporary wafer substrate 442 is then removed, such as by grinding, polishing and etching, with the resulting structure of FIG. 5H, where the piezoresistive layer 436 is exposed.

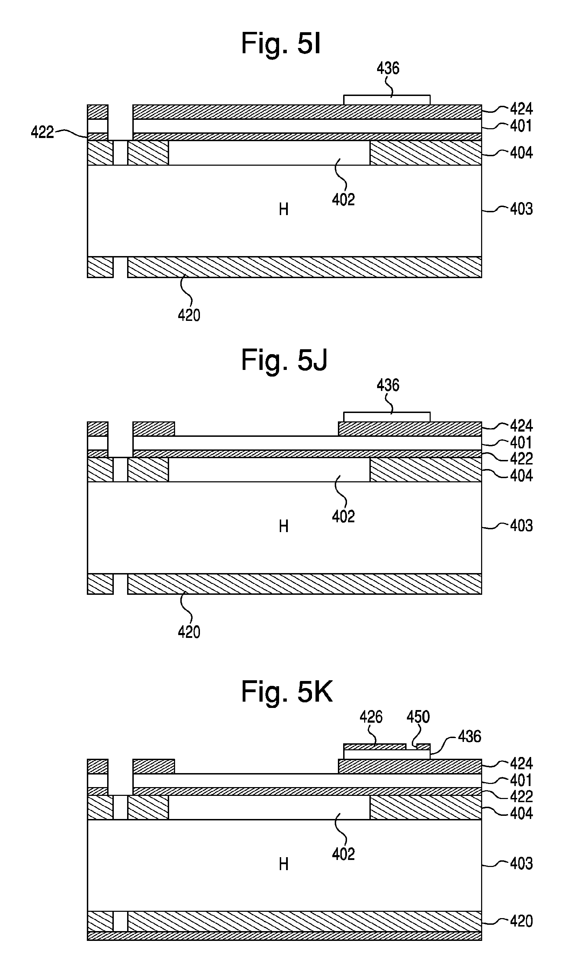

The piezoresistive layer 436 is doped, such as by implanting dopant, and patterned, where the piezoresistive layer 436 in its patterned form is shown in FIG. 5I. The insulator layer 424 is then patterned to expose the surface of the crystalline silicon layer 401 above the diaphragm 406 as shown in FIG. 5J. An insulator layer 426, such as a TEOS oxide layer, is formed and patterned on the piezoresistive layer 436 to expose a portion of the piezoresistive layer 436 through a contact hole 450 in the insulator layer 426 as shown in FIG. 5K. Wiring layer 428 comprising barrier layer 452 of TiW, followed by conductor layer 454 of aluminum, is then deposited and patterned, such that the wiring layer 428 contacts the piezoresistive layer 436 in the contact hole 450 as shown in FIG. 5L.

An overlying insulator layer 460, such as a bilayer of LTO insulator 462 and silicon nitride insulator 464 may be deposited and patterned as shwn in FIG. 5M. The overlying insulator layer 460 is patterned to expose the top surface of diaphragm 406. A passivating layer 430 such as a nitride may be deposited over the patterned wiring layer 428 as shown in FIG. 5N, where a via hole 480 is patterned in the passivating layer 430 and overlying insulator layer 460 down to the wiring layer 428 to provide access for an electrical contact to the wiring layer 428.

Although the present invention has been described with reference to specific embodiments it will be apparent for those skilled in the art that many variations and modifications can be performed within the scope of the invention as described in the specification and defined with reference to the claims below.

## Claims

1. A pressure sensor chip comprising:
a substrate;
a polycrystalline silicon layer formed on the substrate and having a cavity recess formed therein;
at least one silicon layer formed on the polycrystalline silicon layer and covering the cavity recess thereby forming a reference chamber with a diaphragm; and
a diaphragm movement element configured to sense movement of the diaphragm.

2. The sensor chip of claim 1, wherein the cavity recess is formed so as to expose the substrate.

3. The sensor of claim 2, wherein the substrate has an etch stop layer as a top surface, and the cavity recess is formed so as to expose the etch stop layer.

4. The sensor chip of claim 1, wherein the substrate is a semiconductor substrate.

5. The sensor chip of claim 4, wherein the substrate is a silicon substrate.

6. The sensor chip of claim 1, wherein the diaphragm movement element comprises at least one of a piezoresistive element, a capacitor, or a mechanically resonating sensor.

7. The sensor chip of claim 1, wherein the diaphragm movement element comprises at least a piezoresistive layer formed over the silicon layer.

8. The sensor chip of claim 1, wherein the sensor chip is an absolute pressure sensor.

9. The sensor chip of claim 1, wherein the sensor is a differential pressure sensor.

10. The sensor chip of claim 1, wherein the substrate is suitable for processing in silicon standard planar processing.

11. A pressure sensor comprising:
the sensor chip of claim 1, wherein the diaphragm movement element comprises:
a piezoresistive element; and
a temperature sensitive resistor.

12. The sensor of claim 11, wherein the piezoresistive element is part of a first Wheatstone bridge of a sensor circuit, and the temperature sensitive resistor is part of a second Wheatstone bridge of the sensor circuit.

13. The sensor of claim 11, wherein the diaphragm movement element comprises a piezoresistive element formed over the diaphragm, the sensor further comprising:
a group of resistors, the piezoresistive element and the group of resistors in combination forming a Wheatstone bridge.

14. A pressure sensor and guide wire assembly comprising:
a sensor chip comprising:
a substrate;
a polycrystalline silicon layer formed on the substrate and having a cavity recess formed therein;
at least one silicon layer formed on the polycrystalline silicon layer and covering the cavity recess thereby forming a reference chamber with a diaphragm; and
a diaphragm movement element configured to sense movement of the diaphragm;
a wire; and
a mount, wherein the sensor chip is mounted to the wire via the mount.

15. The assembly of claim 14, wherein the cavity recess is formed so as to expose the substrate.

16. The assembly of claim 15, wherein the substrate has an etch stop layer as a top surface, and the cavity recess is formed so as to expose the etch stop layer.

17. The assembly of claim 14, further comprising electrical leads connnected to the pressure sensor.

18. The assembly of claim 14, further comprising a flexible coil.

19. The assembly of claim 14, further comprising a tube surrounding at least a portion of the wire.

20. The assembly of claim 14, wherein the diaphragm movement element comprises:
a piezoresistive element; and
a temperature sensitive resistor.

21. The assembly of claim 14, wherein the diaphragm movement element comprises a piezoresistive element formed over the diaphragm; the assembly further comprising:
a group of resistors, the piezoresistive element and the group of resistors in combination forming a Wheatstone bridge.

22. A method of forming a pressure sensor chip, comprising:
providing a substrate;
forming a polycrystalline silicon layer on the substrate;
forming a cavity recess in the polycrystalline silicon layer;
bonding at least one silicon layer to the polycrystalline silicon layer to cover the cavity recess thereby forming a reference chamber with a diaphragm; and
forming a diaphragm movement element configured to sense movement of the diaphragm.

23. The method of claim 22, wherein the forming a cavity recess comprises forming a cavity recess to expose the substrate.

24. The method of claim 22, wherein providing the substrate comprises providing the substrate with an etch stop layer as a top surface, and wherein the forming the cavity recess comprises forming the cavity recess to expose the etch stop layer.

25. The method of claim 22, wherein the forming a cavity recess comprises etching the polycrystalline silicon layer.

26. The method of claim 25, wherein the forming a cavity recess comprises dry etching the polycrystalline silicon layer.

27. The method of claim 25, wherein the forming a cavity recess comprises wet etching the polycrystalline silicon layer.

28. The method of claim 22, wherein the forming a diaphragm movement element comprises forming at least one of a piezoresistive element, a capacitor, or a mechanically resonating sensor.

29. The method of claim 22, wherein the forming a diaphragm movement element comprises forming a piezoresistive element over the diaphragm.
